# EUROPÄISCHE PATENTANMELDUNG

(11) **EP 3 034 603 A1**
(43) Veröffentlichungstag der Anmeldung: **22.06.2016**
(21) Anmeldenummer: 15003381.9
(22) Anmeldetag: 26.11.2015
(51) Int. Cl.: C12M 1/107, C12M 1/02, C12M 1/00, C12M 1/33

(54) **ERWEITERTE BIOGASANLAGE**

(30) Priorität: 19.12.2014 CZ 20140938
(71) Anmelder: AIVOTEC s.r.o., 76701 Kromeriz (CZ)
(72) Erfinder: Kána, Jan, CZ-76701 Kromeriz (CZ)
(74) Vertreter: Markes, Libor

(57) **Zusammenfassung**

Erweiterte Biogasanlage, die aus einer Einrichtung (1) zur Aufnahme und Aufbereitung der Biomasse, einem Fermenter (2) mit einem Gasbehälter (3), einem Gärrestelager (4) und aus einem Blockheizkraftwerk (5) besteht, ist durch eine Straße zur Bearbeitung von biologisch abbaubarem Abfall ergänzt, die durch eine Einrichtung (6) für Aufnahme und mechanische Zerkleinerung des Abfalls, einen Fermenter (7) für Trocken- oder Halbtrockenfermentation, einen Trockner (9), einen Retortenofen (10) und ein Lager (11) für kohlenstoffhaltige Masse gebildet ist. Dabei ist der Fermenter (7) für Trocken- oder Halbtrockenfermentation an den Gasbehälter (3), ein Abgasaustritt aus dem Blockheizkraftwerk (5) an den Retortenofen (10) und dessen Abgasaustritt durch den Trockner (9) an den Fermenter (7) zu dessen Erwärmung angeschlossen.

## Beschreibung

Die Erfindung betrifft die Erweiterung einer bestehenden landwirtschaftlichen Biogasanlage, die in der Regel aus einer Einrichtung zur Aufnahme und Aufbereitung der Biomasse, einem Fermenter mit einem Gasbehälter, einem Gärrestelager und aus einem Blockheizkraftwerk (BHKW) besteht.

Die Biogasanlagen mit BHKW, die Elektroenergie ins Netz liefern, werden zur Bearbeitung von Standardeinsatzstoffen, d.h. Mais- oder Grassilage mit Mist und Gülle projektiert. Diese konventionellen Biogasanlagen lassen in der Regel keine anderen Substrate, wie biologisch abbaubaren Kommunalmüll zu, da dieser keine feste Zusammensetzung und keinen stabilen Biogasertrag gewährt und inhibierende Beimischungen enthalten kann. Die Biogasanlagen verfügen auch über keine Mittel zur Hygienisierung des Abfalls. Die BHKW der Biogasanlagen erzeugen außer der Elektroenergie auch Wärmeenergie, die mit dem Kühlwasser abgeführt und zum Teil zur Erwärmung des Fermenters genutzt wird. Die in den Abgasen des BHKW enthaltene Wärme bleibt meistens ungenutzt.

Der biologisch abbaubare Kommunalmüll stellt ein bedeutendes energetisches Potenzial dar. Dessen Nutzung limitieren jedoch die mit dem Aufbau und Betrieb der entsprechenden Anlagen verbundenen Kosten. Die Gärrückstände bei der Bearbeitung von Kommunalmüll können nicht als Dünger genutzt werden, so dass deren Entsorgung große Probleme für den Betreiber darstellt.

Zur Bearbeitung von biologisch abbaubarem Abfall bietet sich vorteilhaft die s.g. Trockenfermentation. Typische Fermenter für Trockenfermentation haben die Form gasdichter Kammern mit geheiztem Boden und mit einem Kreislauf durchsickernder Flüssigkeit. Der Fermenter wird mit der zu fermentierenden Biomasse, also der biologisch abbaubaren Substrate verschiedener Herkunft mit 20 % Trockensubstanzgehalt, mittels eines Laders beschickt. Die Beschickung wird ungefähr nach zwei Wochen ausgelagert, zwei Drittel davon werden zur weiteren Fermentation wiederverwendet, und ein Drittel als Gärrest zwischengelagert. Der trockene Gärprozess ist stabil und für energetische Nutzung von beliebigem biologisch abbaubarem Abfall geeignet.

In letzter Zeit wird zur Bearbeitung der biologisch abbaubaren Substrate die s.g. Halbtrockenfermentation eingesetzt - eine anaerobe Fermentation der Beschickung mit bis zu 20 % Trockensubstanzgehalt. Es handelt sich hier um liegende Fermenter mit einem robusten Rührwerk.

Der Erfindung liegt die Aufgabe zu Grunde, eine Anlage an die Hand zu geben, die energetische Nutzung von biologisch abbaubarem Abfall unter akzeptablen Bedingungen ermöglicht.

Erfindungsgemäß wird diese Aufgabe durch eine erweiterte Biogasanlage gelöst, die aus einer Einrichtung zur Aufnahme und Aufbereitung der Biomasse, einem Fermenter mit einem Gasbehälter, einem Gärrestelager und aus einem Blockheizkraftwerk (BHKW) besteht. Die Biogasanlage ist durch eine Straße zur Bearbeitung von biologisch abbaubarem Abfall ergänzt, die durch eine Einrichtung für Aufnahme und mechanische Zerkleinerung des Abfalls, einen Fermenter für Trocken- oder Halbtrockenfermentation, einen Trockner, einen Retortenofen und ein Lager für kohlenstoffhaltige Masse gebildet ist. Der Fermenter für Trocken- oder Halbtrockenfermentation ist dabei an den Gasbehälter, ein BHKW-Abgasaustritt an den Retortenofen und ein Abgasaustritt aus dem Retortenofen durch den Trockner an den Fermenter zu dessen Erwärmung angeschlossen.

Ein Dosierbunker für Gärreste, der auch zum Beimischen anderer Biomassen bestimmt ist, kann vor dem Trockner in die Straße eingebaut werden.

Der Austritt für heiße Kühlflüssigkeit aus dem BHKW kann vorteilhaft an einen Heizkreislauf des Fermenters für Trocken- oder Halbtrockenfermentation angeschlossen sein.

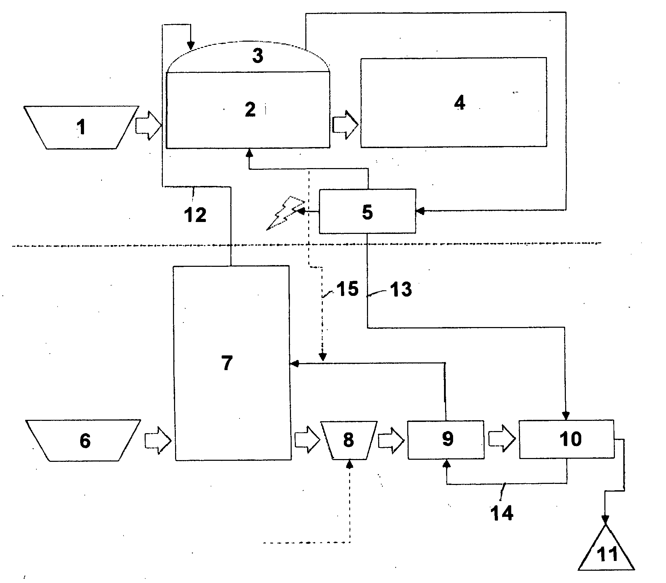

Weitere Einzelheiten der Erfindung werden anhand des in der Zeichnung dargestellten Ausführungsbeispiels näher erläutert. Die Zeichnung zeigt ein Schaltbild einer erweiterten Biogasanlage. Die ursprüngliche konventionelle Biogasanlage ist von der angeschlossenen Straße zur Bearbeitung von biologisch abbaubarem Abfall durch eine Strichpunktlinie getrennt.

Die konventionelle Biogasanlage besteht aus einer Einrichtung **1** zur Aufnahme und Aufbereitung der Biomasse, einem Fermenter **2** mit einem Gasbehälter **3,** einem Gärrestelager **4** und aus einem Blockheizkraftwerk (BHKW) **5.** Die Biogasanlage ist durch eine Straße zur Bearbeitung von biologisch abbaubarem Abfall ergänzt. Diese ist durch eine Einrichtung **6** für Aufnahme und mechanische Zerkleinerung des Bioabfalls, einen Fermenter **7** für Trockenfermentation, einen Dosierbunker **8** für Gärreste, einen Trockner **9,** einen Retortenofen**10** und ein Lager **11** für kohlenstoffhaltige Masse - die Biokohle gebildet. Der obere Teil des Fermenters **7** für Trockenfermentation ist durch eine Gasleitung **12** an den Gasbehälter **3** angeschlossen. Ein BHKW-Abgasaustritt ist durch eine Abgasleitung **13** an den Retortenofen **10** zu dessen Beheizung angeschlossen, wobei die zum Teil abgekühlten Abgase durch eine Leitung **14** durch den Trockner **9** zu einem Wärmeaustauscher im Fermenter **7** für Trockenfermentation weitergeleitet werden. Eine Wasserleitung **15** führt die heiße Kühlflüssigkeit aus dem BHKW **5** dem Fermenter **7** für Trockenfermentation zu.

Die ursprüngliche konventionelle Biogasanlage funktioniert auf eine bekannte Weise. In der angeschlossenen Straße zur Bearbeitung von biologisch abbaubarem Abfall wird der Abfall zuerst in der Einrichtung **6** für Aufnahme und mechanische Zerkleinerung zur Trockenfermentation vorbereitet. Von dort wird er dem Fermenter **7** zugeleitet. Der Fermenter **7** wird semikontinuierlich betrieben. Das durch anaerobe Fermentation erzeugte Gas wird in den Gasbehälter des ursprünglichen Fermenters **2** geleitet. Die aus dem Fermenter **7** abgeführten Gärreste werden im Dosierbunker **8** zwischengelagert, dem man gleichzeitig weitere zur Pyrolyse bestimmte Biomasse zuleiten kann. Vom Dosierbunker **8** gehen die Gärreste in den Trockner **9.** Die ausgetrockneten Gärreste werden in dem Retortenofen **10** verkohlt und als Biokohle im Lager **11** gelagert.

Die für die Pyrolyse notwendige Wärme wird einerseits aus den Abgasen des BHKW **5,** andererseits durch Verbrennung des bei der Pyrolyse entstehenden Gases gewonnen. Das Gemisch der den Retortenofen **10** verlassenden Abgase, die noch ein bedeutendes Wärme-Potenzial haben, wird dann zum Trocknen der Gärreste in dem Trockner **9** und folglich zur Erwärmung der Beschickung des Fermenters **7** für Trockenfermentation abgeleitet.
Das heiße Kühlwasser aus dem BHKW **5** fließt durch die Leitung **15** zur Erwärmung der Beschickung des Fermenters **7** für Trockenfermentation.

Der Betreiber einer konventionellen Biogasanlage wird dem Substrat kaum die biologisch abbaubaren Abfälle beimischen. Solche Abfälle haben keine feste Zusammensetzung und gewähren keinen stabilen Biogasertrag, der für den Betrieb eines BHKW wesentlich ist. Es besteht auch das Risiko, dass die Abfälle Inhibitoren enthalten, die den Gärprozess völlig verhindern. Die vorliegende Erweiterung der bestehenden Biogasanlage erscheint daher als die beste Lösung für energetische und materielle Nutzung von Bioabfall.

Bei der vorliegenden komplexen Bearbeitung von Bioabfall werden auch die Gärreste genutzt, und zwar durch deren Verkohlung. Die erzeugte Biokohle ist als hocheffizienter Bodenverbesserer oder industrieller Rohstoff nutzbar.
Bei der Verkohlung wird sämtliche thermische Restenergie der Abgase des BHKW ausgenutzt. Außerdem ersetzt die Verkohlung den Hygienisierungsprozess von Bioabfall.

Die Kapazität der Anlage sollte der disponiblen Menge von Bioabfall entsprechen, der im Bereich von ca 30 km zur Verfügung steht. Man kann mit 3 bis 5 tausend Tonnen pro Jahr rechnen. Je nach Zusammensetzung des Bioabfalls könnten daraus 200 bis 900 tausend m³ Biogas gewonnen werden. Die Verkohlung solch einer Gärrestmenge bringt 900 bis 1500 Tonnen Biokohle.

Die Effizienz des Betriebs hängt von der festgesetzten Gebühr für die Müllentsorgung, von der Erhöhung der BHKW-Leistung und vom Verkaufspreis für Biokohle ab.

Vorteilhaft ist, dass es sich nur um eine Erweiterung einer bereits bestehenden Biogasanlage handelt, so dass das mit der behördlichen Genehmigung verbundene Verfahren weniger kompliziert wird. Es ist anzunehmen, dass die vorliegende Bearbeitung von Bioabfall das Ablagern von Kommunalabfall bis auf die Hälfte reduzieren kann. Gleichzeitig bietet sich dadurch die Möglichkeit, in der Biogasanlage auch Gastroabfall und Abfall aus der Lebensmittelindustrie zu bearbeiten.

Bei einer günstigen Betriebswirtschaft könnte der Biogasanlagebetreiber den Bioabfall gegen konkurrenzfähige Preise übernehmen, bei bestimmten Abfallarten sogar nur gegen symbolische Gebühren. So könnte ein allgemeines Interesse daran bestehen, Bioabfall vom Stadtmüll zu trennen, was zur Lösung des Problems mit Abfallentsorgung beitragen könnte.

## Patentansprüche

1. Erweiterte Biogasanlage, die aus einer Einrichtung (1) zur Aufnahme und Aufbereitung der Biomasse, einem Fermenter (2) mit einem Gasbehälter (3), einem Gärrestelager (4) und aus einem Blockheizkraftwerk (5) besteht, **dadurch gekennzeichnet, dass** sie durch eine Straße zur Bearbeitung von biologisch abbaubarem Abfall ergänzt ist, die durch eine Einrichtung (6) für Aufnahme und mechanische Zerkleinerung des Abfalls, einen Fermenter (7) für Trocken- oder Halbtrockenfermentation, einen Trockner (9), einen Retortenofen (10) und ein Lager (11) für kohlenstoffhaltige Masse gebildet ist, wobei der Fermenter (7) für Trocken- oder Halbtrockenfermentation an den Gasbehälter (3), ein Abgasaustritt aus dem Blockheizkraftwerk (5) an den Retortenofen (10) und ein Abgasaustritt aus dem Retortenofen durch den Trockner (9) an den Fermenter (7) zu dessen Erwärmung angeschlossen ist.

2. Erweiterte Biogasanlage nach Anspruch 1, **dadurch gekennzeichnet, dass** ein Dosierbunker (8) für Gärreste, der auch zum Beimischen anderer Biomassen bestimmt ist, vor dem Trockner (9) in die Straße eingebaut ist.

3. Erweiterte Biogasanlage nach Anspruch 1 oder 2, **dadurch gekennzeichnet, dass** der Austritt für heiße Kühlflüssigkeit aus dem Blockheizkraftwerk (5) an einen Heizkreislauf des Fermenters (7) für Trocken- oder Halbtrockenfermentation angeschlossen ist.
